# FASCICULE DE BREVET EUROPEEN

(11) **EP 3 997 457 B1**
(45) Date de publication et mention de la délivrance du brevet: **07.06.2023**
(21) Numéro de dépôt: 20750327.7
(22) Date de dépôt: 08.07.2020
(51) Int. Cl.: G01N 33/00

(54) **SYSTÈME DE DÉTECTION POUR NEZ ÉLECTRONIQUE PERMETTANT UNE CLASSIFICATION PHYSICO-CHIMIQUE DES ODEURS ET NEZ ÉLECTRONIQUE COMPRENANT UN TEL SYSTÈME**
DETEKTIONSSYSTEM FÜR EINE ELEKTRONISCHE NASE ZUR PHYSIOCHEMISCHEN KLASSIFIZIERUNG VON GERÜCHEN UND ELEKTRONISCHE NASE MIT EINEM SOLCHEN SYSTEM
DETECTION SYSTEM FOR AN ELECTRONIC NOSE ALLOWING A PHYSICOCHEMICAL CLASSIFICATION OF ODORS AND ELECTRONIC NOSE COMPRISING SUCH A SYSTEM

(30) Priorité: 12.07.2019 FR 1907884
(43) Date de publication de la demande: 18.05.2022
(73) Titulaire: Commissariat à l'énergie atomique et aux énergies alternatives, 75015 Paris (FR); Aryballe, 38000 Grenoble (FR); Centre National de la Recherche Scientifique, 75794 Paris Cedex 16 (FR)
(72) Inventeur: HOU-BROUTIN, Yanxia, 38000 GRENOBLE (FR); BRENET, Sophie, 38000 GRENOBLE (FR); BUHOT, Arnaud, 38000 GRENOBLE (FR); LIVACHE, Thierry, 38000 GRENOBLE (FR); HERRIER, Cyril, 38000 GRENOBLE (FR); ROUSSELLE, Tristan, 38000 GRENOBLE (FR)
(74) Mandataire: Brevalex
(86) Numéro de dépôt international: PCT/FR2020/051212
(87) Numéro de publication internationale: WO 2021/009440

(56) Documents cités:
- JONG HYUN LIM ET AL: "A peptide receptor-based bioelectronic nose for the real-time determination of seafood quality", BIOSENSORS AND BIOELECTRONICS, vol. 39, no. 1, 3 août 2012 (2012-08-03), pages 244-249, XP055686057, AMSTERDAM, NL ISSN: 0956-5663, DOI: 10.1016/j.bios.2012.07.054
- M. DEL CARLO ET AL: "Novel oligopeptides based e-nose for food quality control: application to extra-virgin olive samples", QUALITY ASSURANCE AND SAFETY OF CROPS & FOODS, vol. 6, no. 3, 17 juin 2014 (2014-06-17), pages 309-317, XP055686062, NL ISSN: 1757-8361, DOI: 10.3920/QAS2013.0377

## Description

### Domaine technique

L'invention relève du domaine des nez électroniques.

Plus spécifiquement, l'invention se rapporte à un système de détection pour nez électronique qui permet une classification physico-chimique des odeurs, c'est-à-dire en fonction des propriétés physico-chimiques des composés volatils, ou molécules odorantes, qui composent ces odeurs.

Elle se rapporte également à un nez électronique comprenant un tel système de détection.

L'invention a de nombreuses applications et, notamment, dans :
- le domaine de la santé, par exemple pour offrir un substitut à l'odorat à des personnes souffrant d'une perte de l'odorat, partielle (hyposmie) ou totale (anosmie), ou pour diagnostiquer ou suivre l'évolution de maladies qui s'accompagnent de la présence de composés volatils odorants dans les fluides biologiques ou dans l'haleine, telles que le diabète, certains cancers (cancers de la prostate, du poumon, cancer du poumon, cancer ovarien, ...) et certaines infections microbiennes ;
- les industries alimentaire, cosmétique et pharmaceutique, par exemple pour détecter d'éventuelles contaminations dans les chaînes de fabrication et/ou de distribution ou pour effectuer des contrôles qualité sur les matières premières ou les produits finis ;
- le domaine des arômes et des parfums ;
- le domaine de la sécurité des biens et des personnes, par exemple pour surveiller des sites fabriquant, stockant, manipulant et/ou susceptibles d'être contaminés par des matières volatiles potentiellement dangereuses, pour détecter la présence de substances dangereuses telles que des explosifs ou des agents toxiques, ou de substances illicites telles que des stupéfiants, ou encore pour rechercher des personnes ensevelies sous des décombres ou des éboulis ; et
- le domaine de l'environnement, par exemple pour le suivi de la qualité de l'air ou d'ambiances plus ou moins confinées, ou pour la surveillance et l'analyse de pollutions olfactives d'origine industrielle ou agricole.

### État de la technique antérieure

L'olfaction, ou odorat, est le sens dont sont dotés les mammifères et, notamment, l'homme et qui leur permet de détecter et d'analyser les composés volatils présents dans un milieu gazeux et, notamment, l'air ambiant.

Aujourd'hui, il existe une très forte demande en instruments portables qui soient capables de mimer l'olfaction. Ces instruments sont appelés nez électroniques.

Outre que la détection des composés volatils par un nez électronique doit être rapide et fiable, elle doit de plus être versatile, c'est-à-dire porter sur le plus grand nombre de composés volatils possible. L'objectif n'est pas de réaliser une analyse du milieu gazeux composé volatil par composé volatil, mais de réaliser une discrimination entre les composés volatils présents dans ce milieu qui soit suffisamment fine pour permettre de différencier des odeurs de compositions chimiques très proches.

Aujourd'hui, parvenir à mimer l'olfaction représente toujours un challenge scientifique et technologique car l'olfaction est un sens très complexe qui allie biologie, physicochimie et cognition. Ce sens est resté longtemps peu étudié et son fonctionnement méconnu.

En 1991, L. Buck et R. Axel ont réussi, pour la première fois, à identifier et séquencer des gènes des récepteurs olfactifs (ou OR pour « Olfactive Receptor »). Leurs travaux ont permis d'établir un mécanisme général de fonctionnement de l'olfaction.

Contrairement aux modèles d'interactions usuellement rencontrés en biologie, la détection et l'identification de composés volatils par les OR ne repose pas uniquement sur le modèle de clef-serrure (modèle dans lequel une molécule biologiquement active est reconnue par un ligand qui lui est spécifique, par exemple un anticorps si la molécule biologiquement active est un antigène) mais repose également sur le principe d'une réactivité croisée où :
- chaque OR peut reconnaître plusieurs composés volatils avec différentes affinités ;
- chaque composé volatil peut interagir physico-chimiquement avec plusieurs OR ; et
- différents composés volatils sont reconnus par différentes combinaisons de l'ensemble des OR.

C'est donc en s'inspirant des mécanismes biologiques de l'olfaction qu'ont été conçus les nez électroniques.

Comme son analogue biologique, un nez électronique se compose principalement de trois systèmes, à savoir :
(1) un système fluidique pour le transport d'un échantillon du milieu gazeux entre l'extérieur du nez électronique et l'intérieur de ce nez, ce système jouant le rôle du système respiratoire ;
(2) un système de détection qui comprend un réseau de capteurs (c'est-à-dire d'éléments unitaires de détection) à réactivité croisée vis-à-vis des composés volatils présents dans l'échantillon gazeux, les capteurs jouant le rôle des OR du nez humain ; et
(3) un système informatique de traitement et d'analyse des réponses émises par les capteurs sous forme de signaux, ce système jouant le rôle du cerveau humain.

Au cours des trois dernières décennies, le développement de nez électroniques a connu de grands progrès avec l'utilisation de divers matériaux pour constituer la partie sensible des capteurs (c'est-à-dire la partie des capteurs qui interagit physico-chimiquement avec les composés volatils) et de diverses méthodes de transduction (c'est-à-dire permettant de convertir les interactions physico-chimiques se produisant entre la partie sensible des capteurs et les composés volatils en signaux exploitables).

Cependant, les performances de ces nez électroniques sont encore bien inférieures à celles du nez humain notamment en termes d'aptitude à différencier les odeurs de composition chimique proche.

Dans la plupart des nez électroniques existants, la partie sensible des capteurs, c'est-à-dire celle qui interagit avec les composés volatils, est constituée de matériaux non biologiques tels que des oxydes métalliques semi-conducteurs (ou MOS) et des polymères semi-conducteurs. Ces matériaux ont généralement une sensibilité élevée mais, du fait de la faible variabilité de leurs propriétés physico-chimiques, les mécanismes par lesquels ils interagissent avec les composés volatils (qui sont principalement de type adsorption physique dans le cas des MOS, et de type interactions de Van der Waals et adsorption physique dans le cas des polymères semi-conducteurs) sont trop limités pour permettre une discrimination des odeurs. Au surplus, les MOS présentent l'inconvénient d'avoir une température de fonctionnement élevée (de l'ordre de 200°C à 300°C) tandis que les polymères semi-conducteurs sont sensibles à l'humidité de l'air, ce qui affecte la reproductibilité de la détection.

Pour rendre les nez électroniques plus performants, il a été proposé de réaliser la partie sensible des capteurs avec des molécules organiques, biologiques ou non, de faible masse moléculaire, aux propriétés physico-chimiques variées, qui soient facilement synthétisables et qui puissent, si possible, être auto-assemblées par une ingénierie de surface de sorte à obtenir des films minces, d'épaisseur nanométrique, aux fonctionnalisations reproductibles.

C'est ainsi qu'il a été proposé d'utiliser :
- des molécules d'alcanes terminées par divers groupes chimiques (groupes aromatiques, acides carboxyliques, esters, etc.), ces molécules étant déposées sur des nanotubes de carbone, des nanofils de silicium ou des nanoparticules d'or ;
- des oligonucléotides fixés sur des nanotubes de carbone, du graphène et des nanocristaux de graphène ; et
- des peptides.

Concernant les peptides, on peut citer les travaux de L. A. Beardslee et al. (Proceedings of the IEEE International Conférence on the Micro Electro Mechanical Systems (MEMS) 2011, 964-967, ci-après référence [1]) qui ont utilisé, dans des capteurs résonants à cantilever, trois peptides formés chacun de 23 acides α-aminés et dont une partie sert à immobiliser ces peptides sur des nanotubes de carbone tandis qu'une autre partie est destinée à interagir avec des composés volatils. Cette dernière est composée d'un acide α-aminé répété 7 fois, cet acide α-aminé étant l'arginine dans le premier peptide, l'histidine dans le deuxième et la thréonine dans le troisième. Ces trois acides α-aminés étant polaires et les capteurs étant exposés à de l'éthanol et du toluène, ces auteurs montrent que les peptides et, notamment, celui comportant 8 résidus thréonine ont une plus grande affinité pour l'éthanol, qui est lui-même polaire, que pour le toluène qui est apolaire. Ils en concluent qu'en utilisant une fonctionnalisation par des peptides adaptée, il devrait être possible de moduler l'affinité des capteurs vis-à-vis de composés volatils en fonction de la polarité de ces composés. Ils ne précisent toutefois pas ce que pourrait être cette fonctionnalisation adaptée.

On peut également citer les travaux de D. Compagnone et al. (Biosensors and Bioelectronics 2013, 42, 618-625, ci-après référence [2]) qui ont construit un réseau de capteurs basé sur des nanoparticules d'or fonctionnalisées par l'acide thioglycolique, le glutathion, la cystéine et trois peptides dont deux dipeptides (y-glutamylcystéine et cystéinylglycine) et un hexapeptide. Bien que de différentes longueurs, ces trois peptides possèdent des propriétés physicochimiques proches car ils sont structurellement apparentés au glutathion.

Enfin, il convient de citer les travaux de S. Brenet et al. (Analytical Chemistry 2018, 90, 9879-9887, ci-après référence **[3]**) qui montrent qu'un réseau de capteurs dont les parties sensibles sont fonctionnalisées par des récepteurs constitués de peptides biomimétiques et de molécules thiolées aux propriétés physico-chimiques différentes peut reconnaître de manière discriminante des composés volatils appartenant à différentes classes chimiques (alcools, esters, acides carboxyliques, cétones, hydrocarbures, aldéhydes et amines), offrant ainsi la possibilité de réaliser une différenciation de ces composés sans échelle physico-chimique.

Toutefois, il s'avère que cette référence est totalement silencieuse à la fois sur la composition chimique des peptides biomimétiques et des molécules thiolées formant les récepteurs et que, par voie de conséquence, elle ne permet pas de savoir sur quelle(s) base(s) physico-chimique(s) est obtenue la différentiation des composés volatils.

On peut aussi citer les traveaux de J.H. Lim et al. (Biosensors and Bioelectronics 39 (2013) 244-249, ci après référence [7]).

### Exposé de l'invention

L'invention vise à pallier les insuffisances de l'état de la technique en proposant un système de détection d'un nez électronique qui est apte à détecter et identifier un ensemble E de composés volatils susceptibles d'être présents dans un échantillon gazeux et qui permet une classification physico-chimique de ces composés volatils en fonction de leurs propriétés physico-chimiques.

Ce système de détection comprend une pluralité de capteurs à réactivité croisée vis-à-vis des composés volatils de l'ensemble E, chaque capteur comprenant une partie sensible disposée sur un substrat et fonctionnalisée par un récepteur dont l'interaction physico-chimique avec l'un au moins des composés volatils de l'ensemble E produit un signal détectable, et est caractérisé en ce que :
- il comprend *n* capteurs, *n* étant un nombre entier supérieur ou égal à 3, dont la partie sensible est fonctionnalisée par un récepteur de formule générale (I) :

   X-(Esp)ₘ-Z (I)

   dans laquelle :
   X représente un groupe assurant une immobilisation du récepteur sur la surface du substrat ou le reste d'un composé comprenant un tel groupe,
   m est égal à 0 ou à 1,
   Esp représente un bras espaceur, et
   Z représente une séquence formée par la répétition d'un acide α-aminé ;
- l'acide α-aminé du récepteur d'un capteur d'une première série de capteurs est hydrophile tandis que l'acide α-aminé du récepteur d'un autre capteur de la première série est hydrophobe ;
- les acides α-aminés des récepteurs de deux capteurs d'une deuxième série de capteurs ont des points isoélectriques, respectivement pI₁ et pI₂, qui diffèrent l'un de l'autre d'au moins une unité pH, l'un au moins de ces deux capteurs n'appartenant pas à la première série.

Ainsi, selon l'invention, une classification physico-chimique des composés volatils est obtenue en fonction d'au moins deux de leurs propriétés physico-chimiques, à savoir : d'une part, leur caractère hydrophile ou hydrophobe et, d'autre part, leur caractère acide, neutre ou basique, et ce, en munissant le système de détection du nez électronique d'au moins trois capteurs dont les parties sensibles sont chacune fonctionnalisées par un récepteur comprenant une séquence formée par la répétition d'un acide α-aminé et en choisissant l'acide α-aminé de chaque séquence de sorte que :
- l'acide α-aminé du récepteur d'un premier capteur - qui sera appelé capteur C1 dans ce qui suit - soit hydrophile tandis que l'acide α-aminé du récepteur d'un deuxième capteur - qui sera appelé capteur C2 dans ce qui suit - soit hydrophobe ; et
- les acides α-aminés des récepteurs de deux capteurs aient des points isoélectriques qui diffèrent l'un de l'autre d'au moins une unité pH, l'un de ces deux capteurs pouvant être le capteur C1 ou le capteur C2 mais l'autre de ces capteurs étant un capteur différent des capteurs C1 et C2.

En d'autres termes, le système de détection du nez électronique comprend *a minima :*
- soit trois capteurs, respectivement capteurs C1, C2 et C3, dont la capacité à classer les composés volatils est basée sur deux échelles de propriétés physicochimiques : une échelle d'hydrophilie/hydrophobie à laquelle participent les capteurs C1 et C2, et une échelle d'acidité/basicité à laquelle participent l'un des capteurs C1 et C2 et le capteur C3 ;
- soit quatre capteurs, respectivement capteurs C1, C2, C3 et C4, dont la capacité à classer les composés volatils est également basée sur les deux échelles de propriétés physico-chimiques précitées, les capteurs C1 et C2 participant à la première échelle (hydrophilie/hydrophobie) et les capteurs C3 et C4 participant à la deuxième échelle (acidité/basicité).

Conformément à l'invention, lorsque la séquence Z est formée par la répétition d'un acide α-aminé, alors cet acide α-aminé est, de préférence, choisi parmi les 20 acides α-aminés dits communément « standards », à savoir l'alanine, l'arginine, l'asparagine, l'acide aspartique, la cystéine, l'acide glutamique, la glutamine, la glycine, l'histidine, l'isoleucine, la leucine, la lysine, la méthionine, la phénylalanine, la proline, la sérine, la thréonine, le tryptophane, la tyrosine et la valine, étant entendu, toutefois, que d'autres acides α-aminés tels que la *N*²-acétyllysine, la *N*⁶-acétyllysine, la *N*⁶-méthyllysine, la 5-hydroxylysine, la 4-hydroxyproline, la pyrrolysine, la sélénocystéine, la O-phospho-sérine, la O-phosphothréonine, la O-phosphotyrosine, la norleucine ou la citrulline peuvent également être utilisés.

Par ailleurs, cet acide α-aminé peut être indifféremment de configuration L ou *D* et, dans la mesure où il est présent dans le récepteur sous la forme d'un homopeptide, il peut se trouver dans ce peptide à la fois dans la configuration L et dans la configuration D.

Avantageusement, l'acide α-aminé est répété dans la séquence Z de 1 à 19 fois en sorte que la séquence Z comprend de 2 à 20 fois le même acide α-aminé.

De préférence, l'acide α-aminé est répété dans la séquence Z de 3 à 15 fois en sorte que la séquence Z comprend de 4 à 16 fois le même acide α-aminé.

Mieux encore, l'acide α-aminé est répété dans la séquence Z de 5 à 9 fois en sorte que la séquence Z comprend de 6 à 10 fois le même acide α-aminé.

Conformément à l'invention, l'immobilisation des récepteurs sur le substrat peut être réalisée par l'une quelconque des techniques de fonctionnalisation de surfaces connues de l'homme du métier telles que l'adsorption physique, l'adsorption chimique, le greffage covalent, la synthèse sur substrat, le dépôt de couches minces, l'auto-assemblage moléculaire, etc., étant entendu que le choix de cette technique sera fonction de la nature chimique de la surface du substrat.

X peut donc être tout groupe fonctionnel ou toute fonction chimique (les deux expressions étant considérées comme synonymes) permettant d'immobiliser les récepteurs sur le substrat par l'une de ces techniques ou le reste d'un composé comprenant un ou plusieurs groupes fonctionnels permettant d'immobiliser les récepteurs sur le substrat par l'une de ces techniques. X peut donc comprendre une ou plusieurs fonctions chimiques d'accroche.

À cet égard, on précise que par « reste » ou « partie résiduelle » d'un composé, on entend la partie du composé qui subsiste sur le groupe Esp (si m est égal à 1) ou sur la séquence Z (si m est égal à 0) après sa liaison covalente au groupe Esp ou à la séquence Z.

Ainsi, X peut notamment être un groupe thiol, amine, hydroxyle, carboxyle, nitrile, sélénol, phosphate, sulfonate, silanol, époxy, vinyle, alcyne ou triazide, ou le reste d'un composé comprenant au moins un tel groupe.

Parmi les techniques de fonctionnalisation précitées, préférence est donnée, dans le cadre de l'invention, à l'auto-assemblage moléculaire en raison notamment de sa reproductibilité, auquel cas X peut notamment être :
- un groupe thiol ou le reste d'un composé comprenant, d'une part, au moins un groupe thiol et, d'autre part, un groupe permettant sa liaison covalente au groupe Esp ou à la séquence Z, si la surface du substrat est en or, en platine, en argent, en palladium ou en cuivre, le composé pouvant notamment être la cystéine ou la N-thiométhylglycine ; ou
- un groupe amine ou le reste d'un composé comprenant, d'une part, au moins un groupe amine et, d'autre part, un groupe permettant sa liaison covalente au groupe Esp ou à la séquence Z, si la surface du substrat est en or, en platine, en argent, en palladium ou en cuivre, le composé pouvant notamment être un acide aminé et, notamment, l'un des 20 acides α-aminés standards ; ou
- un groupe silanol ou le reste d'un composé comprenant, d'une part, au moins un groupe silanol et, d'autre part, un groupe permettant sa liaison covalente au groupe Esp ou à la séquence Z, si la surface du substrat est en verre, en quartz, en silicium ou en silice.

Conformément à l'invention, m est, de préférence, égal à 1, ce qui signifie que le groupe Esp est présent, auquel cas ce groupe peut notamment être un groupe hydrocarboné linéaire ou ramifié, saturé ou insaturé, comprenant de 1 à 20 atomes de carbone et éventuellement un ou plusieurs hétéroatomes, cet (ces) hétéroatome(s) étant typiquement choisi(s) parmi l'oxygène, l'azote, le soufre et le silicium.

Le groupe Esp peut ainsi être, par exemple, un groupe alkylène divalent comprenant de 1 à 20 atomes de carbone, de préférence de 1 à 12 atomes de carbone et, mieux encore, de 1 à 6 atomes de carbone, un résidu d'un acide α-aminé ou un enchaînement de résidus d'un ou plusieurs acides α-aminés (jusqu'à concurrence de 20 atomes de carbone).

Selon une disposition particulièrement préférée de l'invention :
- X est le reste d'un acide α-aminé, de préférence la cystéine en raison de son groupe thiol,
- m est égal à 1 et le groupe Esp est un résidu d'un acide aminé, par exemple la glycine, ou un enchaînement de résidus d'un acide α-aminé, par exemple, de résidus de glycine, tandis que
- la séquence Z est une séquence α-peptidique, de préférence hexapeptidique.

Ainsi, les récepteurs sont des α-peptides.

Comme précédemment indiqué, l'acide α-aminé du récepteur du capteur C1 est hydrophile tandis que l'acide α-aminé du récepteur du capteur de C2 est hydrophobe.

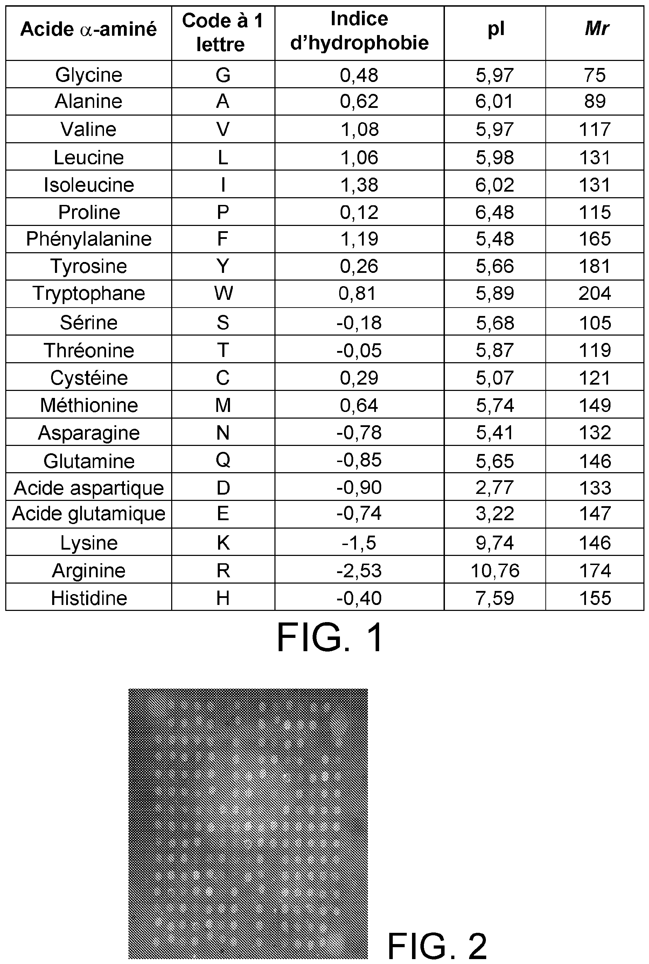

Dans ce qui précède et ce qui suit, on considère qu'un acide α-aminé est hydrophile lorsqu'il présente un indice d'hydrophobie inférieur ou égal à 0,26 selon l'échelle d'hydrophobie de R. M. Sweet et D. Eisenberg (J. Mol. Biol. 1983, 171, 479-488, ci-après référence **[4]**), tandis que l'on considère qu'un acide α-aminé est hydrophobe lorsqu'il présente un indice d'hydrophobie supérieur ou égal à 0,29 selon cette même échelle d'hydrophobie. Les indices d'hydrophobie, tels qu'établis par R. M. Sweet et D. Eisenberg pour les acides α-aminés standards, sont repris dans la figure 1 jointe en annexe.

Ainsi, comme le montre cette figure, la tyrosine, la proline, la thréonine, la sérine, l'histidine, l'asparagine, l'acide glutamique, la glutamine, l'acide aspartique, la lysine et l'arginine sont considérés, dans le cadre de l'invention, comme étant des acides α-aminés hydrophiles tandis que l'isoleucine, la phénylalanine, la valine, la leucine, le tryptophane, la méthionine, l'alanine, la glycine et la cystéine sont considérés comme des acides α-aminés hydrophobes.

Conformément à l'invention, on préfère que les acides α-aminés des récepteurs des capteurs C1 et C2 aient des indices d'hydrophobie relativement éloignés. Par « indices d'hydrophobie relativement éloignés », on entend que la somme des valeurs absolues de ces indices, tels qu'établis par R. M. Sweet et D. Eisenberg, est au moins égale à 1.

Ainsi, par exemple :
- si le récepteur du capteur C1 a, pour acide α-aminé hydrophile, la proline dont l'indice d'hydrophobie est de 0,12 selon l'échelle de R. M. Sweet et D. Eisenberg, alors l'acide α-aminé du récepteur du capteur C2 sera, de préférence, choisi parmi les acides α-aminés dont l'indice d'hydrophobie est au moins égal à 1,06 selon l'échelle de R. M. Sweet et D. Eisenberg (ce qui est le cas de la leucine, de la valine, de la phénylalanine et de l'isoleucine), tandis que
- si le récepteur du capteur C1 a, pour acide α-aminé hydrophile, l'asparagine dont l'indice d'hydrophobie est de -0,74 selon l'échelle de R. M. Sweet et D. Eisenberg, alors l'acide α-aminé du récepteur du capteur C2 pourra être choisi parmi tous les acides α-aminés dont l'indice d'hydrophobie est au moins égal à 0,29 selon l'échelle de R. M. Sweet et D. Eisenberg.

Comme également précédemment indiqué, l'acide α-aminé du récepteur du capteur C3 et l'acide α-aminé du récepteur du capteur C4 ont des points isoélectriques qui diffèrent l'un de l'autre d'au moins une unité pH et, de préférence, d'au moins deux unités pH de sorte à élargir le nombre de composés volatils susceptibles d'être identifiés par le système de détection en fonction de leur caractère acide ou basique.

Les points isoélectriques des acides α-aminés sont indiqués dans de nombreux ouvrages de biochimie et, à défaut, peuvent être déterminés par focalisation isoélectrique (IEF).

À titre d'exemple, la figure 1 jointe en annexe présente les points isoélectriques, notés pl, des acides α-aminés standards tels que repris de l'ouvrage « Lehninger Principles of Biochemistry », chapitre 3, 4ème édition, 2004, ci-après référence **[5].**

Avantageusement, l'acide α-aminé du récepteur d'un capteur d'une troisième série de capteurs est aromatique tandis que l'acide α-aminé du récepteur d'un autre capteur de la troisième série est aliphatique, ces capteurs n'appartenant pas tous les deux à la première série ni à la deuxième série.

En variante ou en complément, les acides α-aminés des récepteurs de deux capteurs d'une quatrième série de capteurs ont des masses moléculaires relatives, respectivement *Mr₁* et *Mr₂,* qui diffèrent l'une de l'autre d'au moins 25 g/mol, de préférence d'au moins 50 g/mol, ces capteurs n'appartenant pas tous les deux à la première série ni à la deuxième série.

Ainsi, la classification physico-chimique des composés volatils par le système de détection du nez électronique peut être obtenue en fonction d'une, voire deux propriétés physico-chimiques additionnelles des composés volatils.

Par « acide α-aminé aromatique », on entend tout acide α-aminé dont la chaîne latérale comprend un groupe aromatique, étant entendu que l'on entend par « groupe aromatique », un groupe qui répond à la règle de Hückel et qui présente donc un nombre d'électrons π délocalisés égal à 4*n* + 2, tandis que par « acide α-aminé ou aliphatique », on entend tout acide α-aminé dont la chaîne latérale est exempte de groupe aromatique tel qu'il vient d'être défini.

Ainsi, par exemple, l'histidine, la phénylalanine, la tyrosine et le tryptophane sont des acides α-aminés aromatiques tandis que l'alanine, l'arginine, l'asparagine, l'acide aspartique, la cystéine, l'acide glutamique, la glutamine, la glycine, l'isoleucine, la leucine, la lysine, la méthionine, la proline, la sérine, la thréonine et la valine sont des acides α-aminés aliphatiques.

À l'instar des points isoélectriques, les masses moléculaires relatives des acides α-aminés sont disponibles dans de nombreux ouvrages de biochimie et, à défaut, peuvent aisément être déterminées à partir des formules chimiques de ces acides α-aminés.

À titre d'exemple, la figure 1 jointe en annexe présente les masses moléculaires relatives, notées *Mr*, des acides α-aminés standards telles que reprises de la référence **[5].**

Si le système de détection du nez électronique comprend la troisième série de capteurs mais sans la quatrième série, alors peuvent appartenir à la troisième série soit deux capteurs, respectivement capteurs C5 et C6, tous deux différents des capteurs C1, C2, C3 et C4, soit l'un des capteurs C5 et C6 conjointement avec l'un des capteurs C1, C2, C3 et C4.

De manière similaire, si le système de détection du nez électronique comprend la quatrième série de capteurs mais sans la troisième série, alors peuvent appartenir à la quatrième série soit deux capteurs, respectivement capteurs C7 et C8, tous deux différents des capteurs C1, C2, C3 et C4, soit l'un des capteurs C7 et C8 conjointement avec l'un des capteurs C1, C2, C3 et C4.

Enfin, si le système de détection du nez électronique comprend à la fois la troisième et la quatrième série de capteurs, alors :
- peuvent appartenir à la troisième série soit les deux capteurs C5 et C6 soit l'un des capteurs C5 et C6 conjointement avec l'un des capteurs C1, C2, C3, C4, C7 ou C8 ; tandis que
- peuvent appartenir à la quatrième série soit les deux capteurs C7 et C8 soit l'un des capteurs C7 et C8 conjointement avec l'un des capteurs C1, C2, C3, C4, C5 ou C6.

Le tableau I ci-après présente les différentes configurations envisageables.

**Tableau I**

| **Nombre de capteurs** | **Échelles physico-chimiques** | | | |
|---|---|---|---|---|
| | **Hydrophilie**/ **hydrophobie** | **Acidité/ basicité** | **Aromaticité/ non aromaticité** | ***Mr*** |
| 3 | C1 et C2 | C3 et (C1 ou C2) | | |
| 4 | C1 et C2 | C3 et C4 | | |
| 4 | C1 et C2 | C3 et (C1 ou C2) | C5 et (C1 ou C2 ou C3) | |
| 5 | C1 et C2 | C3 et (C1 ou C2) | C5 et C6 | |
| 5 | C1 et C2 | C3 et C4 | C5 et (C1 ou C2 ou C3 ou C4) | |
| 6 | C1 et C2 | C3 et C4 | C5 et C6 | |
| 4 | C1 et C2 | C3 et (C1 ou C2) | | C7 et (C1 ou C2 ou C3) |
| 5 | C1 et C2 | C3 et (C1 ou C2) | | C7 et C8 |
| 5 | C1 et C2 | C3 et C4 | | C7 et (C1 ou C2 ou C3 ou C4) |
| 6 | C1 et C2 | C3 et C4 | | C7 et C8 |
| 5 | C1 et C2 | C3 et (C1 ou C2) | C5 et (C1 ou C2 ou C3 ou C7) | C7 et (C1 ou C2 ou C3) |
| 5 | C1 et C2 | C3 et (C1 ou C2) | C5 et (C1 ou C2 ou C3) | C7 et (C1 ou C2 ou C3 ou C5) |
| 6 | C1 et C2 | C3 et (C1 ou C2) | C5 et C6 | C7 et (C1 ou C2 ou C3 ou C5 ou C6) |
| 6 | C1 et C2 | C3 et (C1 ou C2) | C5 et (C1 ou C2 ou C3 ou C7 ou C8) | C7 et C8 |
| 7 | C1 et C2 | C3 et (C1 ou C2) | C5 et C6 | C7 et C8 |
| 6 | C1 et C2 | C3 et C4 | C5 et (C1 ou C2 ou C3 ou C4 ou C7) | C7 et (C1 ou C2 ou C3 ou C4) |
| 6 | C1 et C2 | C3 et C4 | C5 et (C1 ou C2 ou C3 ou C4) | C7 et (C1 ou C2 ou C3 ou C4 ou C5) |
| 7 | C1 et C2 | C3 et C4 | C5 et C6 | C7 et (Clou C2 ou C3 ou C4 ou C5 ou C6) |
| 7 | C1 et C2 | C3 et C4 | C5 et (C1 ou C2 ou C3 ou C4 ou C7 ou C8) | C7 et C8 |
| 8 | C1 et C2 | C3 et C4 | C5 et C6 | C7 et C8 |

Conformément à l'invention, chacun des capteurs que comprend le système de détection peut comprendre son propre système de mesure - ou transducteur - ou partager avec d'autres capteurs un système de mesure qui leur est commun. Dans les deux cas, le système de mesure peut être tout système de mesure permettant de générer un signal exploitable lors de l'interaction physico-chimique entre un composé à l'état gazeux et la partie sensible d'un capteur et peut, notamment, être de type résistif, piézoélectrique, mécanique, acoustique ou optique. En d'autres termes, les capteurs peuvent être des capteurs résistifs, piézo-électriques, mécaniques, acoustiques et/ou optiques.

De façon préférée, les capteurs sont des capteurs optiques à résonance des plasmons de surface, des capteurs interféromètriques ou bien des capteurs à transducteur ultrasonore micro-usiné et, en particulier, des capteurs à transducteur ultrasonore capacitif micro-usiné (ou CMUT) ou à transducteur ultrasonore piézoélectrique micro-usiné (ou PMUT).

Plus encore, on préfère que les capteurs soient des capteurs optiques à résonance des plasmons de surface. Ce type de transduction, qui est connu en soi, combine généralement une source lumineuse, par exemple de type LED, afin de provoquer une excitation plasmonique et une caméra CCD pour enregistrer le signal résultant de la résonance plasmonique. À ce titre, on préfère tout particulièrement que les signaux émis par capteurs soient suivis en mode imagerie qui consiste à suivre les variations de signal de tous les pixels constituant l'image de la caméra CCD utilisée.

Le substrat est constitué d'un matériau adapté au système de mesure. Ainsi, si la mesure est réalisée par résonance des plasmons de surface, alors le substrat comprend, de préférence, un prisme en verre dont une face est recouverte d'une couche métallique, de préférence d'or ou d'argent, typiquement de 10 nm à 100 nm d'épaisseur.

L'invention a également pour objet un nez électronique apte à détecter et identifier un ensemble E de composés susceptibles d'être présents dans un échantillon gazeux, lequel nez électronique est caractérisé en ce qu'il comprend un système de détection tel que précédemment décrit.

Conformément à l'invention, le nez électronique est, de préférence, dédié à la détection et l'identification de composés organiques volatils, du sulfure d'hydrogène (H₂S) et de l'ammoniac (NH₃), ces composés pouvant se trouver seuls ou en mélange dans l'échantillon gazeux.

Dans ce qui précède et ce qui suit, un « composé organique volatil » est défini conformément à la Directive 1999/13/CE du Conseil Européen du 11 mars 1999 en vertu de laquelle :
- un composé organique volatil est « *tout composé organique ayant une pression de vapeur de 0,01 kPa (soit 9,87.10⁻⁵ atm) ou plus à une température de 293,15 K (soit 20°C) ou ayant une volatilité correspondante dans les conditions d'utilisation particulières* » (cf. paragraphe 17 de l'article 2 de la Directive) ;
- un composé organique est « *tout composé comprenant au moins l'élément carbone et un ou plusieurs des éléments suivants : hydrogène, halogènes, oxygène, soufre, phosphore, silicium ou azote, à l'exception des oxydes de carbone et des carbonates et bicarbonates inorganiques* » (cf. paragraphe 16 de l'article 2 de la Directive).

Ainsi, sont notamment considérés comme des composés organiques volatils certains hydrocarbures acycliques, saturés ou insaturés, tels que l'éthane, le propane, le n-butane, le n-hexane, l'éthylène, le propylène, le 1,3-butadiène et l'acétylène, certains hydrocarbures cycliques non aromatiques, saturés ou insaturés, tels que le cyclopropane, le cyclopentane et le cyclohexane, certains hydrocarbures aromatiques tels que le benzène, le toluène, les xylènes et l'éthylbenzène, certains hydrocarbures halogénés tels que le dichlorométhane, le trichlorométhane, le chloroéthane, le trichloroéthylène et le tétrachloroéthylène, certains alcools tels que le méthanol, l'éthanol, le 1-propanol, le 2-propanol, l'éthylène glycol et le propylène glycol, certains aldéhydes tels que le formaldéhyde, l'acétaldéhyde, le propanal et le 2-propenal (ou acroléine), certaines cétones telles que l'acétone, la méthyléthylcétone, la 2-butanone et la méthylvinylcétone, certains esters tels que l'acétate de méthyle, l'acétate d'éthyle, l'acétate d'isopropyle et le butyrate d'isoamyle, certains éthers tels que l'éther diéthylique, le n-butyléther d'éthylène glycol (EGBE) et le 1,4-dioxane, certains acides tels que l'acide acétique et l'acide propanoïque, certaines amines telles que l'éthylamine, la diméthylamine, la triméthylamine, la diéthylamine et l'amylamine, certains amides tels que le diméthylformamide, les composés sulfurés tels que le mercaptan méthylique (ou méthanethiol) et le mercaptan éthylique (ou éthanethiol), et certains nitriles tels que l'acétonitrile et l'acrylonitrile.

D'autres caractéristiques et avantages de l'invention ressortiront du complément de description qui suit, qui se rapporte à des expérimentations ayant permis de valider l'invention et qui est donné en référence aux figures annexées.

Il va de soi toutefois que ce complément de description n'est donné qu'à titre d'illustration de l'objet de l'invention et ne doit en aucun cas être interprété comme une limitation de cet objet.

### Brève description des figures

La figure 1, déjà commentée, est un tableau présentant les indices d'hydrophobie selon l'échelle de R. M. Sweet et D. Eisenberg, les points isoélectriques, notés pl, et les masses moléculaires relatives, notées Mr, des acides α-aminés standards ainsi que leur code à 1 lettre.
La figure 2 est une image différentielle obtenue par imagerie de résonance plasmonique de surface (ou SPRi) qui montre les parties sensibles du réseau de capteurs ayant servi à valider expérimentalement l'invention et dans lequel ces parties sensibles sont agencées sur la surface d'un substrat commun et sont fonctionnalisées par 19 peptides différents, à raison d'un peptide par partie sensible.

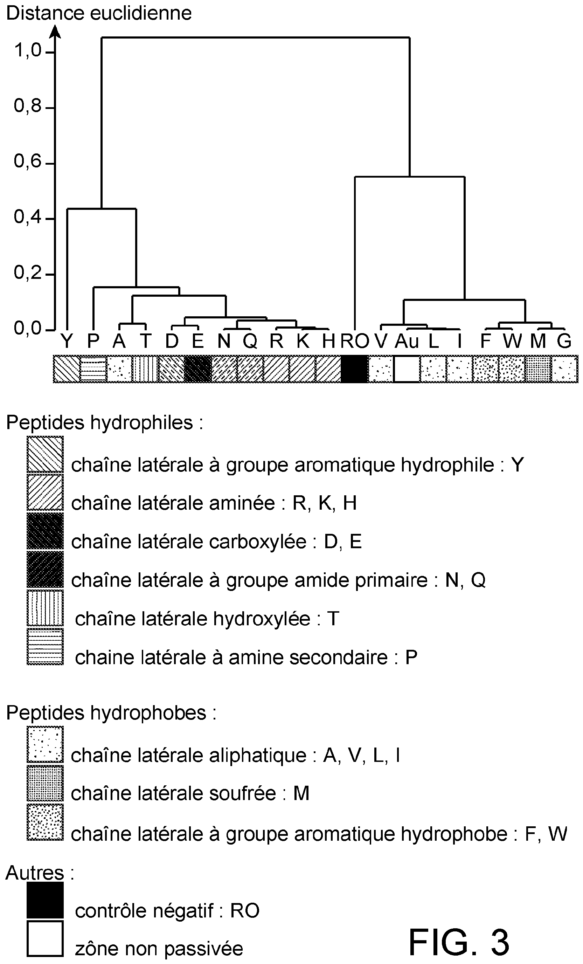

La figure 3 illustre, d'une part, le dendrogramme obtenu par une analyse par classification hiérarchique d'une banque de données constituée à partir des réponses fournies par le réseau de capteurs de la figure 2 lors de tests visant à exposer ce réseau de capteurs à des échantillons gazeux comprenant chacun un composé volatil, et, d'autre part, une légende de ce dendrogramme mettant en correspondance les peptides formant les parties sensibles des capteurs avec le caractère hydrophile ou hydrophobe de l'acide α-aminé formant la séquence Z de ces peptides.

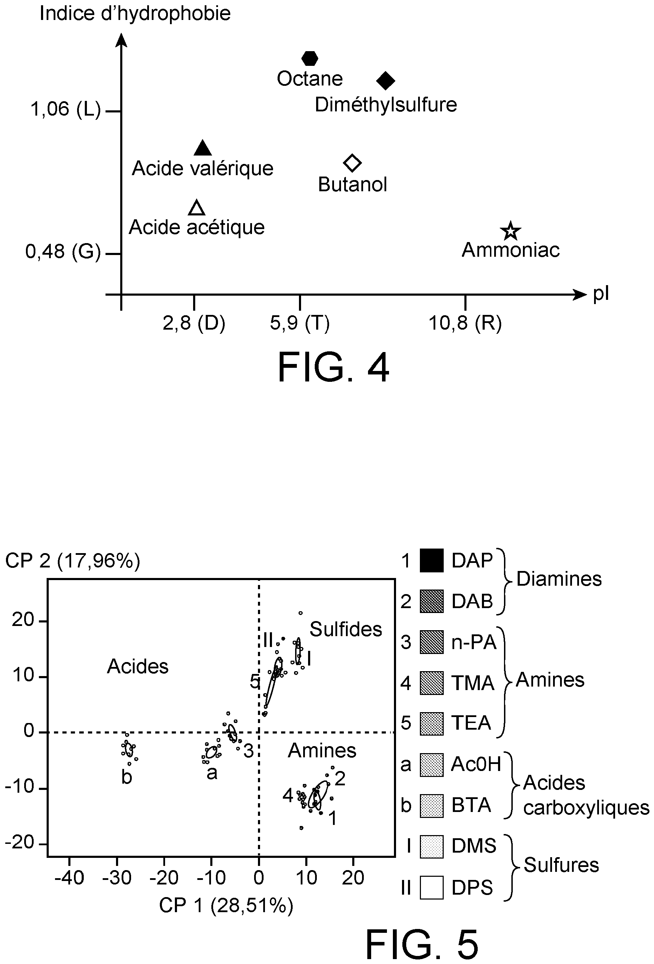

La figure 4 est un graphique illustrant la classification de 6 composés volatils selon deux propriétés physico-chimiques ayant été obtenue à partir d'une banque de données constituée à partir des réponses fournies par le réseau de capteurs de la figure 2 lors de tests visant à exposer ce réseau de capteurs à des échantillons gazeux comprenant chacun un de ces composés volatils ; sur ce graphique, l'axe des abscisses correspond au point isoélectrique, noté pl, des acides α-aminés standards tandis que l'axe des ordonnées correspond à l'indice d'hydrophobie de ces mêmes acides α-aminés.
La figure 5 est la cartographie obtenue par analyse en composantes principales 1 et 2, notées CP 1 et CP 2, d'une banque de données constituée à partir des réponses fournies par le réseau de capteurs de la figure 2 lors de tests visant à exposer ce réseau de capteurs à des échantillons gazeux comprenant chacun un composé volatil ; sur cette figure, les pourcentages de variabilité associés à chacune des CP 1 et CP 2 sont indiqués sur les axes.

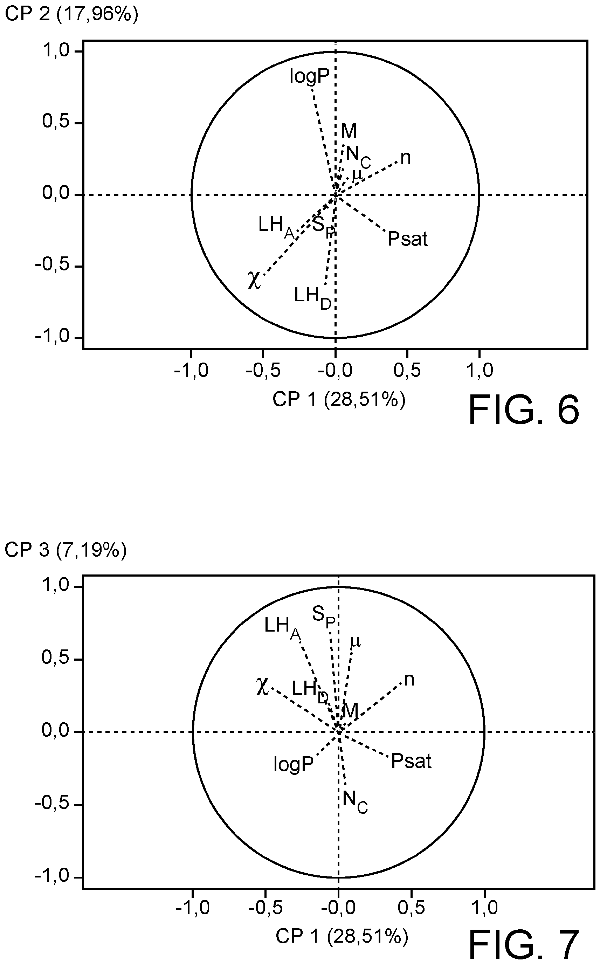

La figure 6 est un premier cercle des corrélations obtenu par analyse en composantes principales 1 et 2, notées CP 1 et CP 2, d'une banque de données constituée à partir des réponses fournies par le réseau de capteurs de la figure 2 lors de tests visant à exposer ce réseau de capteurs à des échantillons gazeux comprenant chacun un composé volatil et corrélation des propriétés physico-chimiques des composés volatils testés avec ces composantes principales.
La figure 7 est un deuxième cercle des corrélations obtenu par analyse en composantes principales 1 et 3, notées CP 1 et CP 3, d'une banque de données constituée à partir des réponses fournies par le réseau de capteurs de la figure 2 lors de tests visant à exposer ce réseau de capteurs à des échantillons gazeux comprenant chacun un composé volatil et corrélation des propriétés physico-chimiques des composés volatils testés avec ces composantes principales.

### Exposé détaillé de modes de réalisation particuliers

L'invention a été validée par les expérimentations décrites ci-après.

Ces expérimentations ont été réalisées en utilisant un nez électronique muni d'un système de détection comprenant :
- d'une part, un réseau de 76 capteurs dont les parties sensibles sont agencées sur un substrat commun et sont fonctionnalisées par des récepteurs peptidiques ; et
- d'autre part, un transducteur optique à résonance des plasmons de surface (ou SPR) commun à tous les capteurs.

A été utilisé comme substrat, un prisme en verre recouvert, sur l'une de ses faces, d'une couche d'or (≈ 50 nm d'épaisseur) tandis qu'ont été utilisés comme récepteurs peptidiques, 19 peptides différents de formule C-G-Z dans laquelle :
- C représente l'acide α-aminé cystéine,
- G représente l'acide α-aminé glycine, et
- Z représente une séquence hexapeptidique constituée de l'un des acides α-aminés suivants : alanine, acide aspartique, acide glutamique, phénylalanine, glycine, histidine, isoleucine, lysine, leucine, méthionine, asparagine, proline, glutamine, arginine, sérine, thréonine, valine, tryptophane et tyrosine.

Ces peptides sont désignés dans ce qui suit ainsi que sur la figure 3 par le code à 1 lettre de l'acide α-aminé formant la séquence Z, ce code étant indiqué dans le tableau de la figure 1.

Ainsi, par exemple, le peptide A correspond au peptide de formule C-G-A-A-A-A-A-A tandis que le peptide T correspond au peptide de formule C-G-T-T-T-T-T-T.

Tous les acides α-aminés entrant dans la constitution des peptides sont de configuration *L*.

Chacune des parties sensibles des capteurs est constituée d'une couche autoassemblée de plusieurs molécules d'un seul et même peptide.

### 1°) Préparation du réseau de capteurs :

Les peptides ont été mis en solution dans du diméthylsulfoxyde (DMSO) à une concentration de 0,1 mmol/L, puis les solutions peptidiques ainsi obtenues ont été déposées sur la couche d'or du substrat au moyen d'un robot microspotteur sans contact (Scienion AG, Allemagne), à raison de quelques nL par partie sensible.

Chaque solution peptidique a été déposée sur 4 zones différentes de la couche d'or du substrat de sorte que chaque capteur soit représenté en quadruple exemplaire, et ce, pour réduire les incertitudes statistiques sur la détection.

Par ailleurs, ont également été créées sur la couche d'or du substrat, également au moyen du robot microspotteur, plusieurs zones de contrôle négatif par dépôt de quelques nL d'une solution comprenant du 1*H*,1*H*,2*H*,2*H*-perfluorodécanethiol dans du DMSO.

Le substrat a ensuite été placé pendant 18 heures dans une chambre fermée pour permettre aux molécules de peptides de s'immobiliser sur la couche d'or de ce substrat par auto-assemblage grâce à la chimie thiol-or (le thiol étant fourni par l'acide α-aminé cystéine des peptides). Puis, le substrat a été rincé pour éliminer les molécules de peptides non immobilisées et, enfin, séché sous un flux d'argon.

A ainsi été obtenu un substrat comprenant 76 parties sensibles susceptibles d'interagir avec des composés volatils ainsi que plusieurs parties de contrôle négatif.

Une image différentielle obtenue par SPRi des parties sensibles du réseau de capteurs, avant toute exposition de ces capteurs à des composés volatils, est illustrée par la figure 2.

### 2°) Vérification de la fonctionnalité du réseau de capteurs :

Le réseau de capteurs a été exposé à une série d'échantillons gazeux comprenant chacun un composé volatil appartenant à l'une des familles chimiques suivantes : alcools, acides carboxyliques, amines, amides, esters, alcanes, aldéhydes, sulfures et composés aromatiques.

Chaque exposition a duré 10 minutes et une purge du système fluidique d'alimentation des capteurs en échantillons gazeux a été systématiquement réalisée entre deux expositions consécutives.

Les interactions entre les parties sensibles des capteurs et les composés volatils ont été suivies en temps réel par SPRi. Les données recueillies par les images SPRi ont été traduites en valeurs de variation de réflectivité, notée Δ%R, qui ont été moyennées pour chaque groupe de 4 capteurs identiques, puis normalisées pour réduire l'impact de la concentration des composés organiques dans les échantillons gazeux sur les données recueillies.

Une banque de données a été constituée à partir des Δ%R ainsi normalisées.

Cette banque de données a été analysée par classification hiérarchique (ou *Hierarchical Clustering* en anglais).

La classification hiérarchique est une méthode d'analyse de données qui consiste à regrouper en classes les données les plus similaires entre elles et à séparer, au contraire, celles qui sont dissemblables en classes distinctes, et ce, en fonction d'un « critère de ressemblance ». Dans le cas présent, le critère de ressemblance correspond aux réponses des capteurs ou, autrement dit, aux interactions s'étant produites entre les parties sensibles des capteurs et les composés volatils auxquels ces capteurs ont été exposés.

Le dendrogramme, ou arbre de classification, résultant de cette analyse est illustré sur la figure 3 ; sur l'axe des abscisses, sont indiqués les peptides formant les parties sensibles du réseau de capteurs (Y, P, A, T, D, E, N, O, R, K, H, V, L, I, F, W, M et G) tandis que, sur l'axe des ordonnées, est indiquée la distance euclidienne existant entre les différentes classes de peptides. Les lettres « RO » et « Au », également présentes sur l'axe des abscisses, correspondent respectivement aux contrôles négatifs et à la zone non passivée du substrat.

En partant du haut de ce dendrogramme, chaque embranchement sépare les peptides en différentes classes, de plus en plus similaires au fur et à mesure que l'on descend dans l'arborescence. Ainsi, les dernières classes formées regroupent les peptides ayant conduit aux réponses des capteurs les plus proches.

Comme le montre le dendrogramme ainsi que la légende de ce dendrogramme, qui est également illustrée sur la figure 3, les peptides à acides α-aminés hydrophiles et les peptides à acides α-aminés hydrophobes sont bien séparés les uns des autres à l'exception de l'alanine tandis que les peptides de même nature chimique sont bien regroupés.

Ces résultats mettent en évidence que les propriétés physico-chimiques des peptides n'ont pas été altérées au cours de la fabrication des réseaux de capteurs.

Ils mettent en évidence que des peptides de même nature chimique ont des interactions similaires avec des composés volatils appartenant à différentes familles chimiques et qu'à l'inverse, des peptides de nature chimique différente ont des interactions différentes avec ces composés. La variété des propriétés physico-chimiques des peptides est donc bien représentée sur le réseau de capteurs et cette variété est à même de permettre une classification absolue des composés volatils selon plusieurs échelles physico-chimiques.

### 3°) Classification des composés volatils selon deux propriétés physicochimiques :

En rapportant les données obtenues au point 2°) ci-avant sur des graphes ayant pour axe des abscisses et axe des ordonnées les valeurs que présentent les acides α-aminés pour deux propriétés physico-chimiques différentes, il a été possible d'obtenir sur ces graphes un ordonnancement des composés organiques en fonction des propriétés physico-chimiques que présentent eux-mêmes ces composés.

Ainsi, par exemple, la figure 4 montre l'ordonnancement obtenu pour 6 composés volatils, à savoir l'acide acétique, l'acide valérique, le butanol, l'ammoniac, le diméthylsulfure et l'octane, sur un graphe dont l'axe des abscisses correspond au point isoélectrique des acides α-aminés et dont l'axe des ordonnées correspond à l'indice d'hydrophobie des acides α-aminés.

Comme visible sur la figure 4, chacun des composés volatils est bien ordonnancé sur le graphe et différenciable en fonction, d'une part, de son caractère acide ou basique (correspondant à l'axe des abscisses) et, d'autre part, de son caractère plus ou moins hydrophobe (correspondant à l'axe des ordonnées).

Une classification absolue des composés volatils en fonction de deux propriétés physico-chimiques est ainsi obtenue.

### 4°) Classification des composés volatils selon plus de deux propriétés physicochimiques :

Pour valider l'aptitude d'un système de détection conforme à l'invention à permettre une classification absolue d'odeurs en fonction d'une grande variété de propriétés physico-chimiques, le réseau de capteurs a été exposé à une série d'échantillons gazeux comprenant chacun l'un des 9 composés volatils suivants :
- acide acétique (CH₃-COOH), noté ci-après AcOH,
- acide butanoïque (CH₃(CH₂)₂COOH), noté ci-après BTA,
- triéthylamine (N(CH₂CH₃)₃), notée ci-après TEA,
- triméthylamine (N(CH₃)₃), notée ci-après TMA,
- n-pentylamine (CH₃(CH₂)₄NH₂), notée ci-après n-PA,
- 1,4-diaminobutane (NH₂(CH₂)₄NH₂), noté ci-après DAB,
- 1,5-diaminopentane (NH₂(CH₂)₅NH₂), noté ci-après DAP,
- diméthylsulfure (S(CH₃)₂), noté ci-après DMS, et
- dipropylsulfure (S(CH₂CH₂CH₃)₂), noté ci-après DPS.

Là également, chaque exposition a duré 10 minutes et une purge du système fluidique d'alimentation des capteurs en échantillons gazeux a été systématiquement réalisée entre 2 expositions consécutives. Les interactions entre les parties sensibles des capteurs et les composés volatils ont été suivies en temps réel par SPRi et les données recueillies par les images SPRi ont été traitées comme précédemment décrit au point 2°) ci-avant.

10 expositions du réseau de capteurs ayant été réalisées par composé volatil, 10 profils ont été obtenus pour chaque composé volatil à partir desquels a été constituée une banque de données.

Cette banque de données a été soumise à une analyse par composantes principales (ou ACP).

L'ACP est une méthode d'analyse factorielle de données couramment utilisée dans le domaine des nez électroniques, notamment pour la réduction des dimensions (cf., par exemple, A. Loufti et al., Journal of Food Engineering 2015, 14, 103-111, ci-après référence **[5]**). Elle permet de représenter un grand nombre de données quantitatives selon une pluralité d'axes factoriels, qui sont des combinaisons linéaires des variables initiales et qui sont appelés « composantes principales » et notés CP 1 pour la composante principale 1, CP 2 pour la composante principale 2, CP 3 pour la composante principale 3, etc., et ce, tout en conservant le maximum d'information.

Appliquée à la banque de données constituée à partir des profils des composés volatils auxquels le réseau de capteurs a été exposé, l'ACP a permis d'obtenir la cartographie de la figure 5 qui représente les profils selon les axes CP 1 et CP 2 et montre un regroupement des profils les plus proches et, notamment, des 10 profils obtenus pour chaque composé volatil, et une séparation des profils éloignés en groupes différents.

Ainsi, par exemple, cette cartographie montre que, si les profils des diamines (DAB et DAP) sont bien regroupés et si les profils des dialkylsulfures (DMS et DPS) sont également bien regroupés, par contre ces derniers sont éloignés des profils des diamines.

Les propriétés physico-chimiques des composés volatils ont ensuite été corrélées avec les composantes CP 1 et CP 2 de la cartographie de la figure 5. Pour ces corrélations, ont été prises en compte :
- le coefficient de partage n-octanol/eau, noté logP et correspondant au rapport des solubilités d'un composé dans ces deux solvants ; il donne une indication du caractère hydrophile ou hydrophobe du composé, le composé étant, en effet, d'autant plus hydrophobe et d'autant moins hydrophile que son logP est élevé ;
- la masse molaire, notée M et exprimée en g/mol ;
- la norme du moment dipolaire, notée µ et exprimé en Debye (D) ; le moment dipolaire est une grandeur vectorielle qui caractérise l'état de polarisation d'une liaison atomique ou d'une molécule ; plus la norme de ce vecteur est grande, plus la molécule est polaire ;
- le nombre de sites donneurs d'une liaison hydrogène, noté LH_{D} ; il s'agit d'atomes d'hydrogène liés à un atome plus électronégatif que l'atome de carbone, susceptibles d'être donnés pour former une liaison hydrogène ;
- le nombre de sites accepteurs d'une liaison hydrogène, noté LH_{A} ; il s'agit d'atomes électronégatifs porteurs de doublets non liants d'électrons, susceptibles de recevoir une liaison hydrogène ;
- la surface polaire, notée S_{P} et exprimée en Å² ; elle est définie comme la somme des surfaces des atomes polaires, comme l'oxygène, l'azote ou le soufre, ainsi que des atomes d'hydrogène liés ;
- la pression de vapeur saturante, notée Pₛₐₜ et exprimée en kPa ;
- l'indice de réfraction du composé pur, noté n ;
- le nombre d'atomes de carbone, noté Ne, que comprend le composé ; et
- la somme des électronégativités, notée Σχ, des hétéroatomes que comprend le composé (χ = 3,5 pour O, χ = 3 pour N ; χ = 2,5 pour S).

Les valeurs de ces propriétés physico-chimiques sont présentées dans le tableau Il ci-après pour chacun des composés volatils.

**Tableau Il**

| **Composé** | **logP** | **M** (g/mol) | **µ** (D) | **LH_{D}** | **LH_{A}** | **S_{P}** (Å²) | **Pₛₐₜ** (kPa) | **n** | **N_{C}** | **Σχ** |
|---|---|---|---|---|---|---|---|---|---|---|
| **AcOH** | -0,2 | 60,052 | 1,7 | 1 | 2 | 37,3 | 1,5 | 1,372 | 2 | 7 |
| **BTA** | 0,79 | 88,106 | 0,93 | 1 | 2 | 37,3 | 0,057 | 1,398 | 4 | 7 |
| **TEA** | 1,4 | 101,193 | 0,66 | 0 | 1 | 3,2 | 8,5 | 1,401 | 6 | 3 |
| **TMA** | 0,3 | 59,112 | 0,612 | 0 | 1 | 3,2 | 187 | 1,3631 | 3 | 3 |
| ***n*-PA** | 1,5 | 87,166 | 1,3 | 1 | 1 | 26 | 3,09 | 1,411 | 5 | 3 |
| **DAB** | -0,9 | 88,154 | 1,26 | 2 | 2 | 52 | 3,1 | 1,457 | 4 | 6 |
| **DAP** | -0,6 | 102,181 | 1,93 | 2 | 2 | 52 | 0,09 | 1,458 | 5 | 6 |
| **DMS** | 2,7 | 150,298 | 1,56 | 0 | 1 | 25,3 | 0,86 | 1,4487 | 6 | 2,5 |
| **DPS** | 0,9 | 62,13 | 1,45 | 0 | 1 | 25,3 | 57 | 1,435 | 2 | 2,5 |

A ainsi été obtenu le cercle des corrélations montré sur la Figure 6.

À cet égard, on rappelle qu'un cercle des corrélations est un cercle de rayon 1 dont les deux diamètres perpendiculaires représentent deux composantes principales, CP 1 et CP 2 dans le cas de la figure 6. Les descripteurs - c'est-à-dire les paramètres physico-chimiques des composés volatils dans le cas de la figure 6 - sont disposés dans ce cercle et leur projection sur les composantes principales donnent leur coefficient de corrélation avec ces composantes.

Ainsi, la projection du logP sur CP 2 est d'environ 0,8, ce qui signe une corrélation positive entre ce paramètre physico-chimique et CP 2. Comme la corrélation est positive, alors plus le logP d'un composé volatil est élevé, plus la coordonnée de ce composé sur CP 2 est haute. On trouve donc les composés volatils les plus hydrophobes en haut de la cartographie de la figure 5 et les plus hydrophiles en bas de cette cartographie. Le réseau de capteurs est donc capable de différencier les composés volatils en fonction de leur caractère hydrophile ou hydrophobe et de les classer sur une échelle d'hydrophilie/ hydrophobie.

De manière similaire, la figure 6 montre que le nombre LH_{D} de sites donneurs d'une liaison hydrogène est également corrélé avec CP 2. Le réseau de capteurs est donc également capable de différencier les composés volatils en fonction de leur capacité à former une liaison hydrogène et de les classer sur une échelle de pH.

La figure 7 illustre un autre de cercle des corrélations obtenu en corrélant les paramètres physico-chimiques des composés volatils avec la composante CP 1 de la cartographie de la figure 5 et une composante CP 3, également obtenue par l'analyse ACP mais non représentée sur cette cartographie. Cette figure montre que la norme µ du moment dipolaire ainsi que la surface polaire S_{P} sont bien corrélées avec CP 3, ce qui signifie que le réseau de capteurs est capable de différencier les composés volatils en fonction de leur polarité et de les classer sur une échelle de polarité.

### Références

**[1]** L. A. Beardslee et al., Proceedings of the IEEE International Conférence on the Micro Electro Mechanical Systems (MEMS) 2011, 964-967
**[2]** D. Compagnone et al., Biosensors and Bioelectronics 2013, 42, 618-625
**[3]** S. Brenet et al., Analytical Chemistry 2018, 90, 9879-9887
**[4]** R. M. Sweet et D. Eisenberg, J. Mol. Biol. 1983, 171, 479-488
**[5]** Lehninger Principles of Biochemistry, chapitre 3, 4ème édition, 2004
**[6]** A. Loufti et al., Journal of Food Engineering 2015, 14, 103-111
[7] JONG HYUN LIM ET AL: "A peptide receptor-based bioelectronic nose for the real-time détermination of seafood quality", BIOSENSORS AND BIOELECTRONICS, vol. 39, no. 1, 2012-08-03, pages 244-249, XP055686057, ISSN: 0956-5663, DOI: 10.1016/j.bios.2012.07.054

## Revendications

1. Système de détection d'un nez électronique apte à détecter et identifier un ensemble E de composés volatils susceptibles d'être présents dans un échantillon gazeux, lequel système de détection comprend *n* capteurs à réactivité croisée vis-à-vis des composés volatils de l'ensemble E, *n* étant un nombre entier supérieur ou égal à 3, chaque capteur comprenant une partie sensible disposée sur un substrat et fonctionnalisée par un récepteur dont l'interaction physico-chimique avec l'un au moins des composés volatils de l'ensemble E produit un signal détectable, et est **caractérisé en ce que** :
- la partie sensible des capteurs est fonctionnalisée par un récepteur de formule générale (I) :
X-(Esp)ₘ-Z (I)
dans laquelle :
X représente un groupe fonctionnel assurant une immobilisation du récepteur sur la surface du substrat ou le reste d'un composé comprenant au moins un tel groupe,
m est égal à 0 ou 1,
Esp représente un bras espaceur, et
Z représente une séquence formée par la répétition d'un acide α-aminé ;
- l'acide α-aminé du récepteur d'un capteur d'une première série de capteurs est hydrophile tandis que l'acide α-aminé du récepteur d'un autre capteur de la première série est hydrophobe ;
- les acides α-aminés des récepteurs de deux capteurs d'une deuxième série de capteurs ont des points isoélectriques, respectivement pI₁ et pI₂, qui diffèrent l'un de l'autre d'au moins une unité pH, l'un au moins de ces deux capteurs n'appartenant pas à la première série.

2. Système de détection selon la revendication 1, dans lequel pI₁ et pI₂ diffèrent l'un de l'autre d'au moins deux unités pH.

3. Système de détection selon la revendication 1 ou la revendication 2, dans lequel la séquence Z est formée par la répétition d'un acide α-aminé choisi parmi l'alanine, l'arginine, l'asparagine, l'acide aspartique, la cystéine, l'acide glutamique, la glutamine, la glycine, l'histidine, l'isoleucine, la leucine, la lysine, la méthionine, la phénylalanine, la proline, la sérine, la thréonine, le tryptophane, la tyrosine ou la valine.

4. Système de détection selon l'une quelconque des revendications 1 à 3, dans lequel l'acide α-aminé est répété dans Z de 1 à 19 fois, de préférence de 3 à 15 fois, mieux encore de 5 à 9 fois.

5. Système de détection selon la revendication 4, dans lequel X est un groupe thiol, amine, hydroxyle, carboxyle, nitrile, sélénol, phosphate, sulfonate, silanol, époxy, vinyle, alcyne ou triazide, ou le reste d'un composé comprenant au moins un groupe thiol, amine, hydroxyle, carboxyle, nitrile, sélénol, phosphate, sulfonate, silanol, époxy, vinyle, alcyne ou triazide.

6. Système de détection selon l'une quelconque des revendications 1 à 5, dans lequel m vaut 1 et Esp est un groupe hydrocarboné linéaire ou ramifié, saturé ou insaturé, comprenant de 1 à 20 atomes de carbone et éventuellement un ou plusieurs hétéroatomes.

7. Système de détection selon l'une quelconque des revendications 1 à 6, dans lequel X est le reste d'un acide α-aminé, m est égal à 1, Esp comprend un résidu d'un acide α-aminé ou un enchaînement de résidus d'un acide α-aminé, et Z est une séquence α-peptidique.

8. Système de détection selon la revendication 7, dans lequel X est le reste d'une cystéine et Z est une séquence hexapeptidique.

9. Système de détection selon l'une quelconque des revendications 1 à 8, dans lequel l'acide α-aminé hydrophile et l'acide α-aminé hydrophobe ont des indices d'hydrophobie selon l'échelle d'hydrophobie de R. M. Sweet et D. Eisenberg dont la somme des valeurs absolues est au moins égale à 1.

10. Système de détection selon l'une quelconque des revendications 1 à 9, dans lequel l'acide α-aminé du récepteur d'un capteur d'une troisième série de capteurs est aromatique tandis que l'acide α-aminé du récepteur d'un autre capteur de la troisième série est aliphatique, ces capteurs n'appartenant pas tous les deux à la première série ni à la deuxième série.

11. Système de détection selon l'une quelconque des revendications 1 à 9, dans lequel les acides α-aminés des récepteurs de deux capteurs d'une quatrième série de capteurs ont des masses moléculaires relatives qui diffèrent l'une de l'autre d'au moins 25 g/mol, de préférence d'au moins 50 g/mol, ces capteurs n'appartenant pas tous les deux à la première série ni à la deuxième série.

12. Système de détection selon l'une quelconque des revendications 1 à 11, dans lequel les capteurs sont des capteurs résistifs, piézo-électriques, mécaniques, acoustiques et/ou optiques.

13. Système de détection selon la revendication 12, dans lequel les capteurs sont des capteurs optiques à résonance des plasmons de surface ou interférométriques, de préférence à transducteur ultrasonore micro-usiné.

14. Nez électronique apte à détecter et identifier un ensemble E de composés susceptibles d'être présents dans un échantillon gazeux, **caractérisé en ce qu'**il comprend un système de détection tel que défini dans l'une quelconque des revendications 1 à 13.

15. Nez électronique selon la revendication 14, dans lequel les composés de l'ensemble E sont des composés organiques volatils, le sulfure d'hydrogène et l'ammoniac.

## Patentansprüche

1. Detektionssystem einer elektronischen Nase, das geeignet ist, eine Gruppe E von flüchtigen Verbindungen, die in einer Gasprobe vorhanden sein können, zu detektieren und zu identifizieren, wobei das Detektionssystem n Sensoren mit Kreuzreaktivität gegenüber flüchtigen Verbindungen der Gruppe E umfasst, wobei n eine ganze Zahl größer oder gleich 3 ist, wobei jeder Sensor einen empfindlichen Teil umfasst, der auf einem Substrat angeordnet und durch einen Rezeptor funktionalisiert ist, dessen physikalisch-chemische Wechselwirkung mit mindestens einer der flüchtigen Verbindungen der Gruppe E ein detektierbares Signal erzeugt, und **dadurch gekennzeichnet ist, dass**:
- der empfindliche Teil der Sensoren funktionalisiert ist mit einem Rezeptor der allgemeinen Formel (I):
X-(Esp)ₘ-Z (I)
wobei:
X eine funktionelle Gruppe darstellt, die eine Immobilisierung des Rezeptors auf der Oberfläche des Substrats gewährleistet, oder den Rest einer Verbindung, die mindestens eine solche Gruppe umfasst,
m 0 oder 1 ist,
Esp einen Abstandshalterarm darstellt, und
Z eine Sequenz darstellt, die durch die Wiederholung einer α-Aminosäure gebildet wird;
- die α-Aminosäure des Rezeptors eines Sensors aus einer ersten Serie von Sensoren hydrophil ist, während die α-Aminosäure des Rezeptors eines anderen Sensors aus der ersten Serie hydrophob ist;
- die α-Aminosäuren der Rezeptoren von zwei Sensoren aus einer zweiten Serie von Sensoren isoelektrische Punkte, jeweils pI₁ und. pI₂, aufweisen, die sich um mindestens eine pH-Einheit voneinander unterscheiden, wobei mindestens einer dieser beiden Sensoren nicht zu der ersten Serie gehört.

2. Detektionssystem nach Anspruch 1, wobei sich pI₁ und pI₂ um mindestens zwei pH-Einheiten voneinander unterscheiden.

3. Detektionssystem nach Anspruch 1 oder 2, wobei die Sequenz Z durch die Wiederholung einer α-Aminosäure gebildet wird, die ausgewählt ist aus Alanin, Arginin, Asparagin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Tyrosin oder Valin.

4. Detektionssystem nach einem der Ansprüche 1 bis 3, wobei die α-Aminosäure in Z 1- bis 19-mal, vorzugsweise 3- bis 15-mal, besonders bevorzugt 3-bis 9-mal, wiederholt wird.

5. Detektionssystem nach Anspruch 4, wobei X eine Thiol-, Amin-, Hydroxyl-, Carboxyl-, Nitril-, Selenol-, Phosphat-, Sulfonat-, Silanol-, Epoxid-, Vinyl-, Alkin- oder Triazidgruppe oder der Rest einer Verbindung ist, die mindestens eine Thiol-, Amin-, Hydroxyl-, Carboxyl-, Nitril-, Selenol-, Phosphat-, Sulfonat-, Silanol-, Epoxid-, Vinyl-, Alkin- oder Triazidgruppe enthält.

6. Detektionssystem nach einem der Ansprüche 1 bis 5, wobei m für 1 steht und Esp eine lineare oder verzweigte, gesättigte oder ungesättigte Kohlenwasserstoffgruppe ist, die 1 bis 20 Kohlenstoffatome und gegebenenfalls ein oder mehrere Heteroatome umfasst.

7. Detektionssystem nach einem der Ansprüche 1 bis 6, wobei X der Rest einer α-Aminosäure ist, m gleich 1 ist, Esp einen Rest einer α-Aminosäure oder eine Kette von Resten einer α-Aminosäure umfasst und Z eine α-Peptidsequenz ist.

8. Detektionssystem nach Anspruch 7, wobei X der Rest eines Cysteins ist und Z eine Hexapeptidsequenz ist.

9. Detektionssystem nach einem der Ansprüche 1 bis 8, wobei die hydrophile α-Aminosäure und die hydrophobe α-Aminosäure Hydrophobie-Indizes nach der Hydrophobie-Skala von R.M. Sweet und D. Eisenberg aufweisen, deren Summe der absoluten Werte mindestens 1 beträgt.

10. Detektionssystem nach einem der Ansprüche 1 bis 9, wobei die α-Aminosäure des Rezeptors eines Sensors aus einer dritten Serie von Sensoren aromatisch ist, während die α-Aminosäure des Rezeptors eines anderen Sensors aus der dritten Serie aliphatisch ist, wobei diese Sensoren nicht beide zu der ersten Serie oder zu der zweiten Serie gehören.

11. Detektionssystem nach einem der Ansprüche 1 bis 9, wobei die α-Aminosäuren der Rezeptoren von zwei Sensoren einer vierten Serie von Sensoren relative Molekulargewichte aufweisen, die sich um mindestens 25 g/mol, vorzugsweise um mindestens 50 g/mol, voneinander unterscheiden, wobei diese Sensoren nicht beide zu der ersten Serie oder zu der zweiten Serie gehören.

12. Detektionssystem nach einem der Ansprüche 1 bis 11, wobei es sich bei den Sensoren um resistive, piezoelektrische, mechanische, akustische und/oder optische Sensoren handelt.

13. Detektionssystem nach Anspruch 12, wobei die Sensoren optische Oberflächenplasmonenresonanz- oder interferometrische Sensoren sind, vorzugsweise mit mikrobearbeitetem Ultraschallwandler.

14. Elektronische Nase, die in der Lage ist, eine Gruppe E von Verbindungen, die in einer Gasprobe vorhanden sein können, zu detektieren und zu identifizieren, **dadurch gekennzeichnet, dass** sie ein Detektionssystem umfasst, wie es in einem der Ansprüche 1 bis 13 definiert ist.

15. Elektronische Nase nach Anspruch 14, wobei es sich bei den Verbindungen der Gruppe E um flüchtige organische Verbindungen, Schwefelwasserstoff und Ammoniak handelt.

## Claims

1. Detection system for an electronic nose capable of detecting and identifying a set E of volatile compounds likely to be present in a gaseous sample, which detection system comprises n sensors with cross-reactivity relative to the volatile compounds of the set E, n being an integer greater than or equal to 3, each sensor comprising a sensitive part disposed on a substrate and functionalised by a receptor whose physicochemical interaction with at least one of the volatile compounds of the set E produces a detectable signal, and is **characterised in that**:
- the sensitive part of the sensors is functionalised by a receptor of general formula (I):
X-(Esp)ₘ-Z (I)
wherein:
X represents a functional group ensuring an immobilisation of the receptor on the surface of the substrate or the residue of a compound comprising at least one such group,
m is equal to 0 or 1,
Esp represents a spacer arm, and
Z represents a sequence formed by the repetition of an α-amino acid;
- the α-amino acid of the receptor of a sensor of a first series of sensors is hydrophilic while the α-amino acid of the receptor of another sensor of the first series is hydrophobic;
- the α-amino acids of the receptors of two sensors of a second series of sensors have isoelectric points, respectively pl1 and pI2, which differ from each other by at least one pH unit, at least one of these two sensors not belonging to the first series.

2. Detection system according to claim 1, wherein pI1 and pI2 differ from each other by at least two pH units.

3. Detection system according to claim 1 or claim 2, wherein the sequence Z is formed by the repetition of an α-amino acid selected from alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine or valine.

4. Detection system according to any one of claims 1 to 3, wherein the α-amino acid is repeated in Z from 1 to 19 times, preferably from 3 to 15 times, more preferably from 5 to 9 times.

5. Detection system according to claim 4, wherein X is a thiol, amine, hydroxyl, carboxyl, nitrile, selenol, phosphate, sulphonate, silanol, epoxy, vinyl, alkyne or triazide group, or the residue of a compound comprising at least one thiol, amine, hydroxyl, carboxyl, nitrile, selenol, phosphate, sulphonate, silanol, epoxy, vinyl, alkyne or triazide group.

6. Detection system according to any one of claims 1 to 5, wherein m is 1 and Esp is a linear or branched, saturated or unsaturated hydrocarbon group comprising 1 to 20 carbon atoms and optionally one or more heteroatoms.

7. Detection system according to any one of claims 1 to 6, wherein X is the residue of an α-amino acid, m is equal to 1, Esp comprises a residue of an α-amino acid or a chain of residues of an α-amino acid, and Z is an α-peptide sequence.

8. Detection system according to claim 7, wherein X is the residue of a cysteine and Z is a hexapeptide sequence.

9. Detection system according to any one of claims 1 to 8, wherein the hydrophilic α-amino acid and the hydrophobic α-amino acid have hydrophobicity indices according to the hydrophobicity scale of R.M. Sweet and D. Eisenberg whose sum of absolute values is at least equal to 1.

10. Detection system according to any one of claims 1 to 9, wherein the α-amino acid of the receptor of a sensor of a third series of sensors is aromatic while the α-amino acid of the receptor of another sensor of the third series is aliphatic, these sensors not both belonging to the first series nor to the second series.

11. Detection system according to any one of claims 1 to 9, wherein the α-amino acids of the receptors of two sensors of a fourth series of sensors have relative molecular weights which differ from each other by at least 25 g/mol, preferably at least 50 g/mol, these sensors not both belonging to the first series nor to the second series.

12. Detection system according to any one of claims 1 to 11, wherein the sensors are resistive, piezoelectric, mechanical, acoustic and/or optical sensors.

13. Detection system according to claim 12, wherein the sensors are surface plasmon resonance or interferometric optical sensors, preferably with a micromachined ultrasonic transducer.

14. Electronic nose capable of detecting and identifying a set E of compounds likely to be present in a gaseous sample, **characterised in that** it comprises a detection system as defined in any one of claims 1 to 13.

15. Electronic nose according to claim 14, wherein the compounds of the set E are volatile organic compounds, hydrogen sulphide and ammonia.
